# EUROPÄISCHE PATENTSCHRIFT

(11) **EP 3 741 338 B1**
(45) Veröffentlichungstag und Bekanntmachung des Hinweises auf die Patenterteilung: **06.09.2023**
(21) Anmeldenummer: 20170165.3
(22) Anmeldetag: 17.04.2020
(51) Int. Cl.: A61F 13/20

(54) **TAMPON**
PAD
TAMPON

(30) Priorität: 24.05.2019 DE 202019102949 U
(43) Veröffentlichungstag der Anmeldung: 25.11.2020
(73) Patentinhaber: H Y G I E N E Oederan Produktionsgesellschaft mbH Herstellung von Damen- und Erwachsenenhygiene, 09569 Oederan (DE)
(72) Erfinder: Kunick, Paul, 09648 Altmittweida (DE); Lothar, Schmidt, 56727 Mayen (DE)
(74) Vertreter: Steiniger, Carmen

(56) Entgegenhaltungen:
- EP-A1- 0 564 681
- EP-A2- 2 236 110
- DE-A1- 19 753 665
- US-A- 4 056 103
- US-A1- 2005 113 786

## Beschreibung

Die vorliegende Erfindung betrifft einen Tampon mit einem absorbierenden, aus wenigstens einem überwiegend aus Viskose und/oder Baumwolle bestehenden und keine thermoplastischen Bestandteile aufweisenden Kardenband ausgebildeten Tamponkern und wenigstens einem wenigstens einem thermoplastische Fasern und Viskose- und/oder Baumwollfasern aufweisenden, um den Tamponkern vorgesehenen Hüllvlies.

Die Druckschrift EP 0 564 681 A1 beschreibt einen Tampon mit einem gewickelten Kern aus einem Faservlies und einem den Kern umgebenden Mantel. Der Mantel besteht aus einem Faservlies, das neben thermoplastischen Fasern hydrophile Fasern aufweist, die beim Feuchtwerden erweichen, wodurch sich der Tamponmantel eng um den Tamponkern schmiegt.

Die Druckschrift DE 197 53 665 A1 offenbart einen Tampon, der aus einem kompressiblen Tamponrohling aus einem flüssigkeitsabsorbierenden Material besteht, der zumindest teilweise von einer Umhüllung umgeben ist. Die Umhüllung, die auf die Endform des Tampons gepresst wird, besteht aus einem flüssigkeitsdurchlässigen Vlies aus wirr liegenden, thermoplastischen und heißsiegelfähigen Fasern, wobei das Vlies durch eine Behandlung mit Hitze und Druck geglättet wurde.

Die Druckschrift US 4 056 103 A beschreibt eine verbesserte Umhüllungsstruktur für Tampons, die als ein flüssigkeitsdurchlässiges Netz um einen Kern ausgebildet ist, der superabsorbierendes Material enthält. Die Umhüllungsstruktur weist eine hohe Rückhaltekapazität für Feuchtigkeit auf, um den hohen Kapillarsaugdruck der superabsorbierenden Fasern im Tamponkern zumindest teilweise zu kompensieren. Damit wird eine flüssigkeitsgeschmierte Oberfläche des Tampons gewahrt, die das Herausziehen des gebrauchten Tampons erleichtert.

Die Druckschrift EP 2 236 110 A2 beschreibt ein aus zwei Schichten bestehendes Hüllvlies für Tampons. Dabei besteht die dem Tamponkern zugewandte Schicht aus einem nach einem Trockenverfahren gebildeten Vliesstoff. Die zweite Schicht, welche die äußere Oberfläche des Tampons bildet, besteht aus einem Extrusionsvliesstoff, der eine über die gesamte Tamponoberfläche hinweg gleichmäßige Flüssigkeitsaufnahme gewährleisten soll.

In der Druckschrift US 2005/113786 A1 wird ein Tampon mit Rillen beschrieben, die in Längsrichtung des Tampons in Ihrer Breite variieren. Die durch die Rillen vergrößerte Tamponoberfläche verbessert die Aufnahmerate bei Vorliegen einer größeren Flüssigkeitsmenge.

Aus der aus dem Jahr 1970 stammenden Druckschrift DE 2 053 040 A ist ein Tampon bekannt, der einen aus einer oder mehreren Zellwoll- oder Zellwoll-Baumwoll-Faserschichten gewickelten und zusammengepressten Tamponkörper aufweist, um welchen ein bindemittelfreies Zellwoll- oder Zellwoll-Baumwoll-Hüllvlies vorgesehen ist, das die Aufgabe hat, den Tamponkörper auch im vollgesaugten Zustand zusammenzuhalten und zu verhindern, dass sich einzelne Fasern ablösen und in der Körperhöhle verbleiben können. Als Alternativen zu dem Hüllvlies sind ein an der Oberfläche des Tampons vorgesehener Strickschlauch oder eine durchlöcherte Gummi- oder Kunststoffhülle angegeben. Um einen solchen Tampon herstellen zu können, muss das Hüllvlies beispielsweise schlauchförmig vorgefertigt und dann über den Tamponkörper gezogen werden. Zudem hat es sich gezeigt, dass insbesondere das Einführen solcher Tampons aufgrund ihrer rauen Oberfläche problematisch und sogar schmerzhaft sein kann.

In der Druckschrift DE 25 20 899 A1 aus dem Jahr 1975 ist ein als Tampon zu verwendender Saugkörper ohne Hülle beschreiben. Der Saugkörper besteht aus hydrophilen Saugstoff-Fasern, die in Form eines dreidimensionalen Netzes mit geeigneten Bindemitteln, wie z.B. thermoplastischen Klebstoffen in Pulver- oder Faserform, abgebunden und dadurch verfestigt sind. Im Zentrum des Saugkörpers ist ein langgestreckter Kern aus steifem Material vorgesehen, an dem eine Zugschnur befestigt ist. Um eine ausreichende Stabilität dieses Tampons zu gewährleisten, ist im gesamten Saugkörper ein entsprechend hoher Thermoplastgehalt vorzusehen, was sich andererseits jedoch nachteilig auf die Saugeigenschaften des Saugkörpers auswirkt.

In der im Jahr 1976 angemeldeten Patentschrift DE 26 25 177 C3 ist ein für Damenbinden, Schritteinlagen, Windeln, Krankenunterlagen, Schuheinlagen oder Achseleinlagen, aber nicht für den Einsatz als Tampon beschriebener Saugkörper offenbart. Der Saugkörper ist im Wesentlichen plattenförmig aus wirr gelegten hydrophilen Fasern ausgebildet. An den Saugkörper schmiegt sich eine Umhüllung völlig dicht und faltenlos an, wobei die Umhüllung praktisch kontinuierlich in den eigentlichen Saugkörper übergeht und dadurch fest mit diesem verbunden ist. Die Umhüllung ist durch auf der Oberfläche des Saugkörpers vorgesehene Thermoplastpartikel ausgebildet, deren Dichte zum Saugkörper-Inneren hin abnimmt.

Auf diese Weise entsteht ein Konzentrationsgradient des Bindemittels, der eine gleichmäßige Verankerung der verfestigten Oberflächenschicht im Saugkörper-Inneren garantiert. Dabei reichen hydrophile Fasern aus der Umhüllungsschicht bis in das Innere des Saugkörpers, wodurch sie nach Art eines Dochtes die außen anfallende Flüssigkeit nach innen leiten. Um solche Saugkörper herzustellen, werden zuerst zwei Formhälften mit einer Schicht aus hydrophilen Hüllvliesfasern und thermoplastischem Bindemittel bedeckt, die Formhälften dann mit Zellstoff-Flocken gefüllt und im Nachgang die gefüllten Formhälften zusammengepresst und daraufhin unter Wärmeeinwirkung nachverdichtet. Eine solche Vorgehensweise ist zur Herstellung von Tampons ungeeignet.

Um einen Tampon mit hoher Saugwirkung und dennoch hoher Stabilität auf effiziente Weise herstellen zu können, ist man, wie in der Druckschrift DE 20 2009 004 264 U1 beschrieben, später dazu übergegangen, um einen aus Viskose oder einer Viskose-Baumwoll-Mischung ausgebildeten, absorbierenden Tamponkern, in den typischerweise ein Rückholfaden eingearbeitet ist, ein Hüllvlies aus synthetischen Fasern vorzusehen. Als Hüllvliesfasern kommen beispielsweise Stapelfasern aus Polyethylen im Mantel und Polypropylen oder Polyethylenterephthalat im Kern zum Einsatz.

Durch die synthetischen Fasern kann einerseits das Hüllvlies mittels einer Heißversiegelung fest an dem Tamponkern angebracht werden. Andererseits ergibt sich durch die synthetischen Fasern die Möglichkeit, eine glattere Tamponoberfläche auszubilden, wodurch insbesondere das Einführen des Tampons erleichtert wird.

Da synthetische Fasern jedoch nur eine geringe Saugkraft aufweisen, wurden in das Hüllvlies Perforationen eingearbeitet, die ein Durchdringen von Flüssigkeit in den saugfähigen Tamponkern erlauben. Damit besitzen solche Tampons eine hohe Saugkraft.

Diese Saugkraft führt jedoch gerade zu Beginn und am Ende eines Menstruationszyklus dazu, dass die Scheide stark ausgetrocknet wird, was einen Tamponwechsel erschwert.

Es ist daher die Aufgabe der vorliegenden Erfindung, einen Tampon zur Verfügung zu stellen, der leicht einführbar ist, gut Körperflüssigkeiten aufnimmt, nicht ausfasert oder sich auflöst und darüber hinaus jederzeit leicht ausführbar ist.

Die Aufgabe wird durch einen Tampon der eingangs angegebenen Gattung gelöst, bei dem die Viskose- und/oder Baumwollfasern unmodifizierte Viskose- und/oder Baumwollfasern sind und der Anteil der Viskose- und/oder Baumwollfasern in dem Hüllvlies wenigstens 3 Gew.-% und weniger als 10 Gew.-%, vorzugsweise von 4 Gew.-% bis 9 Gew.-% beträgt.

Bei dem erfindungsgemäßen Tampon bilden die Viskose- und/oder Baumwollfasern in dem auf der Oberfläche des Tampons vorhandenen Hüllvlies Feuchtigkeitsspeicher, welche auch dann, wenn der Tampon wenig Flüssigkeit aufgenommen hat und von wenig Flüssigkeit umgeben ist, eine Art Gleitlager ausbilden, durch die der Tampon leicht ausführbar ist. Durch das Vorsehen der Viskose- und/oder Baumwollfasern in dem Hüllvlies erhält das Hüllvlies eine Hydrophilie. Die beim Gebrauch des Tampons feuchten Viskose- und/oder Baumwollfasern in dem Hüllvlies wirken also als Schmierung, das heißt als Schmierfasern.

Da das Hüllvlies neben den Viskose- und/oder Baumwollfasern auch thermoplastische Fasern aufweist, ist es trotzdem siegelfähig und glatt, sodass einerseits eine gute Stabilität des Tampons garantiert werden kann und andererseits ein leichtes Einführen des Tampons im trockenen Zustand gewährleistet ist. Das Hüllvlies verhindert eine Desintegration des Tampons, insbesondere bei dessen Ausführen.

Die thermoplastischen Fasern können beispielsweise Bikomponentenfasern sein.

Obwohl bei der vorliegenden Erfindung der Anteil von Viskose- und/oder Baumwollfasern in dem Hüllvlies mit wenigstens 3 Gew.-% und weniger als 10 Gew.-%, vorzugsweise zwischen 4 Gew.-% und 9 Gew.-%, sehr gering ist, reicht dieser Anteil aus, um eine geeignete Gleitlagerwirkung, wie oben beschrieben, hervorzurufen. Bei dieser Konzentration wirken sich die Viskose- und/oder Baumwollfasern nicht negativ auf die Rauhigkeit der Tamponoberfläche und damit auf die Einführbarkeit des Tampons im trockenen Zustand und auch nicht auf die Versiegelbarkeit des Hüllvliesmaterials aus.

Die vorliegende Erfindung geht damit einen Weg, der der Entwicklungsrichtung des Standes der Technik regelrecht entgegen gerichtet ist. Während man im Stand der Technik, wie bei Windeln oder Inkontinenzeinlagen, gerade jede Form von Rücknässe auf der Oberfläche des jeweiligen Hygieneproduktes vermeiden will, werden bei der vorliegenden Erfindung Viskose- und/oder Baumwollfasern in dem Hüllvlies vorgesehen, die bei Tampongebrauch in jedem Fall feucht werden und auch bleiben.

Vorzugsweise besteht das Hüllvlies nur aus thermoplastischen Fasern und Viskose- und/oder Baumwollfasern, weist also keine weiteren Fasern auf.

Das überwiegend aus Viskose und/oder Baumwolle bestehenden und keine thermoplastischen Bestandteile aufweisenden Kardenband, aus dem der Tamponkern ausgebildet ist, besteht zu über 50 Gew.-%, vorzugsweise zu wenigstens 75 Gew.-%, besonders bevorzugt zu wenigstens 90 Gew.-% aus Viskose und/oder Baumwolle, insbesondere Viskose- und/oder Baumwollfasern.

Vorzugsweise befindet sich in dem Tamponkern und in dem Hüllvlies jeweils der gleiche Gew.-%-Anteil von Viskose- und/oder Baumwollfasern. Damit weisen trotz des bei dem Hüllvlies vorhandenen Lubrikationseffektes der Tamponkern und das Hüllvlies die gleiche Saugfähigkeit auf.

In vorteilhaften Ausbildungen des erfindungsgemäßen Tampons ist ein erster Endbereich des Hüllvlieses an dem wenigstens einen Kardenband befestigt, das Hüllvlies um den Tamponkern gewickelt und ein dem ersten Endbereich gegenüber befindlicher zweiter Endbereich des Hüllvlieses mit dem Hüllvlies verschweißt. Das Hüllvlies wird also mit sich selbst versiegelt und bildet somit eine Tamponhülle.

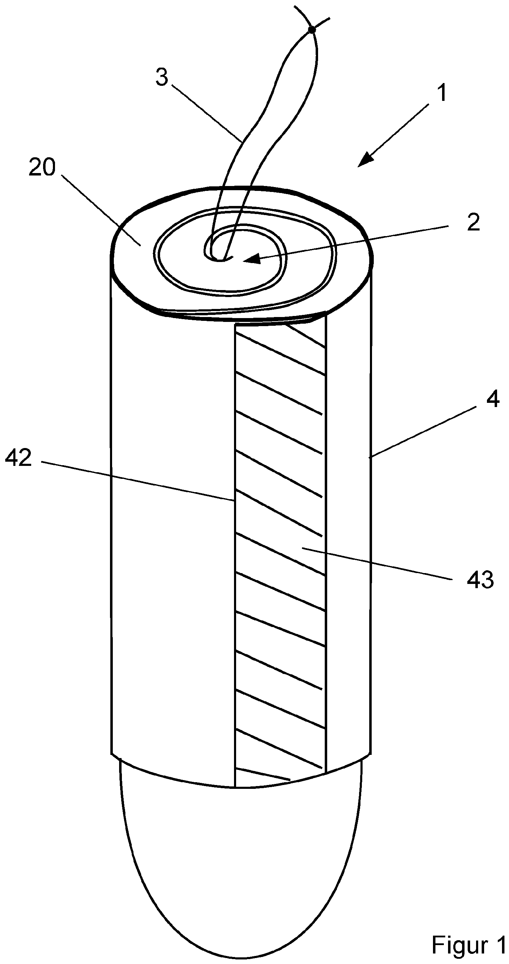

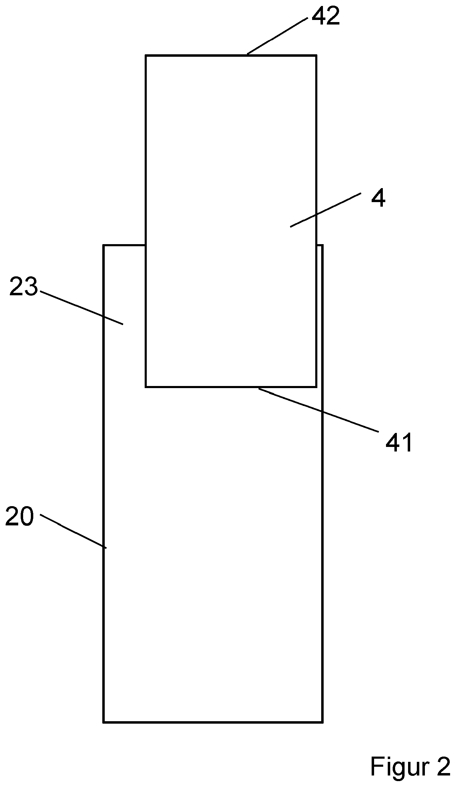

Eine bevorzugte Ausführungsform der vorliegenden Erfindung wird im Folgenden anhand der Figuren 1 und 2 näher erläutert. Dabei zeigen
- Figur 1: eine Ausführungsform eines erfindungsgemäßen Tampons von der Seite mit Blick auf dessen Unterseite; und
- Figur 2: eine Draufsicht auf ein noch nicht aufgewickeltes Kardenband mit einem daran befestigten, noch nicht aufgewickelten Hüllvlies zur Ausbildung eines erfindungsgemäßen Tampons.

Figur 1 zeigt eine Ausführungsform eines erfindungsgemäßen Tampons 1. Der Tampon 1 weist einen zylindrischen Tamponkern 2 auf. Der Tamponkern 2 besteht in dem vorliegenden Ausführungsbeispiel zu mindestens 95 %, vorzugsweise 99 % aus einem Viskose- und/oder Baumwoll-Gemisch. Das Viskose- und/oder Baumwoll-Gemisch ist in Form eines zusammengerollten Wattebandes oder Kardenbandes 20 ausgebildet. Unter dem Watteband oder Kardenband 20 wird in der vorliegenden Erfindung ein kardiertes Wirrvlies verstanden. Das Watteband oder Kardenband 20 kann beispielsweise mittels eines Dorns aufgewickelt sein. In das aufgewickelte Kardenband 20 ist in dem gezeigten Ausführungsbeispiel ein Rückholfaden 3 eingebunden.

Um den Tamponkern 2 ist ein Hüllvlies 4 gewunden. Das Hüllvlies 4 weist sowohl thermoplastische Fasern 5 als auch Viskose- und/oder Baumwollfasern 6 auf.

Die Viskose- und/oder Baumwollfasern 6 sind in dem gezeigten Ausführungsbeispiel unmodifizierte Fasern. Das heißt, diese Fasern sind keine gekräuselten Fasern, weisen keine wasserabweisende Beschichtung, keine Farbbeschichtung und keine flammhemmende Beschichtung auf.

Das Hüllvlies 4 weist beispielsweise einen Anteil von thermoplastischen Fasern 5 in einem Bereich von mehr als 90 Gew.-% und höchstens 97 Gew.-%, vorzugsweise von 91 Gew.-% bis 96 Gew.-% auf. Das Hüllvlies 4 weist einen Anteil von Viskose- und/oder Baumwollfasern 6 in einem Bereich von 3 Gew.-%, aber weniger als 10 Gew.-%, vorzugsweise von 4 Gew.-% bis 9 Gew.-% auf.

Figur 2 zeigt eine Draufsicht auf das Kardenband 20 und das Hüllvlies 4 vor deren Aufwicklung zu dem Tampon 1. In Figur 2 liegen das Kardenband 20 und das Hüllvlies noch in flächiger Form vor. Wie in Figur 2 zu sehen, ist ein erstes Hüllvliesende 41 mit einer Oberfläche 23 des Kardenbandes 20 verbunden, vorzugsweise verschweißt.

Wie in Figur 1 zu sehen, ist das Hüllvlies 4 so um den Tamponkern 2 gewickelt, dass es in einem Überlappungsbereich 43 auf sich selbst liegt, wobei ein dem ersten Hüllvliesende 41 gegenüber liegendes zweites Hüllvliesende 42 das erste Hüllvliesende 41 überlappt und mit dem Material des Hüllvlieses 4 verschweißt ist. Unter einem Verschweißen wird in der vorliegenden Erfindung ein Versiegeln unter Wärme- und/oder Druckeinwirkung verstanden.

## Patentansprüche

1. Tampon (1) mit einem absorbierenden, aus wenigstens einem überwiegend aus Viskose und/oder Baumwolle bestehenden und keine thermoplastischen Bestandteile aufweisenden Kardenband (20) ausgebildeten Tamponkern (2) und wenigstens einem thermoplastische Fasern (5) und Viskose- und/oder Baumwollfasern (6) aufweisenden, um den Tamponkern (2) vorgesehenen Hüllvlies (4),
**dadurch gekennzeichnet,**
**dass** die Viskose- und/oder Baumwollfasern (6) unmodifizierte Viskose- und/oder Baumwollfasern sind und der Anteil der Viskose- und/oder Baumwollfasern (6) in dem Hüllvlies (4) wenigstens 3 Gew.-% und weniger als 10 Gew.-% beträgt.

2. Tampon nach Anspruch 1, **dadurch gekennzeichnet, dass** der Anteil der Viskose- und/oder Baumwollfasern (6) in dem Hüllvlies (4) zwischen wenigstens 4 Gew.-% und 9 Gew.-% beträgt.

3. Tampon nach Anspruch 1 oder 2, **dadurch gekennzeichnet, dass** das Hüllvlies (4) nur aus thermoplastischen Fasern (5) und Viskose- und/oder Baumwollfasern (6) besteht.

4. Tampon nach wenigstens einem der vorhergehenden Ansprüche, **dadurch gekennzeichnet, dass** sich in dem Tamponkern (2) und in dem Hüllvlies (4) jeweils der gleiche Gew.-%-Anteil von Viskose- und/oder Baumwollfasern befindet.

5. Tampon nach wenigstens einem der vorhergehenden Ansprüche, **dadurch gekennzeichnet, dass** ein erster Endbereich (41) des Hüllvlieses (4) an dem wenigstens einen Kardenband (20) befestigt ist, das Hüllvlies (4) um den Tamponkern (2) gewickelt ist und ein dem ersten Endbereich (41) gegenüber befindlicher zweiter Endbereich (42) des Hüllvlieses (4) mit dem Hüllvlies (4) verschweißt ist.

## Claims

1. A tampon (1) with an absorbent tampon core (2) formed from at least one card sliver (20) consisting predominantly of viscose and/or cotton and having no thermoplastic constituents, and at least one enveloping non-woven (4) provided around the tampon core (2) and having thermoplastic fibers (5) and viscose and/or cotton fibers (6),
**characterized in that**
viscose and/or cotton fibers (6) are unmodified viscose and/or cotton fibers and the proportion of viscose and/or cotton fibers (6) in the enveloping non-woven (4) is at least 3% by weight and less than 10% by weight.

2. The tampon according to claim 1, **characterized in that** the proportion of viscose and/or cotton fibers (6) in the enveloping non-woven (4) is between at least 4% and 9% by weight.

3. The tampon according to claim 1 or 2, **characterized in that** the enveloping non-woven (4) consists of thermoplastic fibers (5) and viscose and/or cotton fibers (6) only.

4. The tampon according to at least any of the preceding claims, **characterized in that** the tampon core (2) and the enveloping non-woven (4) each contain the same proportion by weight of viscose and/or cotton fibers.

5. The tampon according to at least any of the preceding claims, **characterized in that** a first end portion (41) of the enveloping non-woven (4) is fastened to the at least one card sliver (20), the enveloping non-woven (4) is wound around the tampon core (2), and a second end portion (42) of the enveloping non-woven (4) located opposite the first end area (41) is fused to the enveloping non-woven (4).

## Revendications

1. Tampon (1) comportant un coeur de tampon (2) absorbant formé d'au moins un ruban cardé (20) constitué principalement de viscose et/ou de coton et ne présentant pas de composants thermoplastiques, et au moins un intissé enveloppant (4) présentant des fibres thermoplastiques (5) et des fibres de viscose et/ou de coton (6) et prévu autour du coeur de tampon (2),
**caractérisé en ce**
**que** les fibres de viscose et/ou de coton (6) sont des fibres de viscose et/ou de coton non modifiées et la proportion des fibres de viscose et/ou de coton (6) dans l'intissé enveloppant (4) est d'au moins 3 % en poids et de moins de 10 % en poids.

2. Tampon selon la revendication 1, **caractérisé en ce que** la proportion des fibres de viscose et/ou de coton (6) dans l'intissé enveloppant (4) est comprise entre au moins 4 % en poids et 9 % en poids.

3. Tampon selon la revendication 1 ou 2, **caractérisé en ce que** l'intissé enveloppant (4) est constitué uniquement de fibres thermoplastiques (5) et de fibres de viscose et/ou de coton (6).

4. Tampon selon au moins l'une des revendications précédentes, **caractérisé en ce que,** dans le coeur de tampon (2) et dans l'intissé enveloppant (4), se trouve respectivement la même proportion en % en poids de fibres de viscose et/ou de coton.

5. Tampon selon au moins l'une des revendications précédentes, **caractérisé en ce qu'**une première région d'extrémité (41) de l'intissé enveloppant (4) est fixée à l'au moins un ruban cardé (20), l'intissé enveloppant (4) est enroulé autour du coeur de tampon (2) et une seconde région d'extrémité (42) de l'intissé enveloppant (4) se trouvant à l'opposé de la première région d'extrémité (41) est soudée à l'intissé enveloppant (4).
